(19) Europäisches Patentamt
European Patent Office
Office européen des brevets

(11) Veröffentlichungsnummer: **0 015 308**
**B1**

(12) # EUROPÄISCHE PATENTSCHRIFT

(45) Veröffentlichungstag der Patentschrift: **29.04.87**

(51) Int. Cl.⁴: **C 12 P 33/00**, C 07 J 9/00, C 12 N 15/00

(21) Anmeldenummer: **79104165.0**

(22) Anmeldetag: **29.10.79**

(54) **Verfahren zum selektiven Seitenkettenabbau von Steroidverbindungen und zur Herstellung hierzu geeigneter Mikroorganismen-Defektmutanten sowie neue Mikroorganismenstämme (II).**

(30) Priorität: **07.03.79 AT 1709/79**

(43) Veröffentlichungstag der Anmeldung: **17.09.80 Patentblatt 80/19**

(45) Bekanntmachung des Hinweises auf die Patenterteilung: **29.04.87 Patentblatt 87/18**

(84) Benannte Vertragsstaaten: **DE FR GB IT NL**

(56) Entgegenhaltungen:
**EP-A-0 004 913**

**CHEMICAL ABSTRACTS, Band 86, Nr. 3, 17. Januar 1977, Seite 304, Nr. 15144z, Columbus, Ohio, USA, C.K.A. MARTIN et al.: "Microbial transformation of beta-sitosterol by Nocardia sp. M 29."**
**CHEMICAL ABSTRACTS, Band 87, Nr. 23, 5. Dezember 1977, Seite 464, Nr. 182618j, Columbus, Ohio, USA**
**BIOCHIMICA ET BIPHYSICA ACTA, Band 531, 1978, Elsevier, Amsterdam, NL., M.G. WOVCHA et al.: "Bioconversion of sitosterol to useful steroidal intermediates by mutants of mycobacterium fortuitum", Seiten 308-320**
**AGRICULTURAL AND BIOLOGICAL CHEMISTRY, Band 42/2, Februar 1978, Seiten 411-416, Tokyo, JP., KEI ARIMA et al.: "Microbial production of 3.Oxobisnorchola-1,4-dien-22-oic Acid"**

(73) Patentinhaber: **Henkel Kommanditgesellschaft auf Aktien, Postfach 1100 Henkelstrasse 67, D-4000 Düsseldorf- Holthausen (DE)**

(72) Erfinder: **Hill, Frank, Dr., Schlesienstrasse 23, D-4020 Mettmann- Obschwarzbach (DE)**
Erfinder: **Preuss, Wolfgang, Dr., Finkenweg 12, D-4019 Monheim (DE)**
Erfinder: **Schindler, Joachim, Dr., Am Eichelkamp 158, D-4010 Hilden (DE)**
Erfinder: **Schmid, Rolf, Dr., Am Nettschesfeld 30, D-4000 Düsseldorf 13 (DE)**
Erfinder: **Struve, Alfred, Dr., Am Eichelkamp 53, D-4010 Hilden (DE)**

(74) Vertreter: **Werner, Hans- Karsten, Dr., Deichmannhaus am Hauptbahnhof, D-5000 Köln 1 (DE)**

(56) Entgegenhaltungen: (Fortsetzung)

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 015 308 B1

LIBER, STOCKHOLM 1987

**Beschreibung**

Es ist bekannt, daß zahlreiche Mikroorganismenstämme befähigt sind, auf Sterinverbindungen pflanzlichen oder tierischen Ursprungs zu wachsen. Solche Sterinverbindungen natürlichen Ursprungs, wie Cholesterin, Sitosterin, Stigmasterin und dergleichen, enthalten in 17-C-Substitution eine längere Alkyl- bzw. Alkenyl-Seitenkette. Beim Wachstum der in der Natur vorkommenden Wildstämme auf Sterinverbindungen als Kohlenstoffquelle werden in der Regel in nicht spezifischer Weise sowohl das Ringsystem als auch eventuell vorhandene Seitenketten am Ringsystem durch die Mikroorganismen angegriffen bzw. bei ihrem Wachstum abgebaut. Überwiegend sind dabei Angriff und Abbau im Ringsystem die bevorzugte und/oder schnellere Reaktion.

Der Stand der Technik hat mehrfach Vorschläge entwickelt, eine selektive Abbauleistung zu erzwingen, durch die ein Abbau des Seitenkettensubstituenten in C-17 ermöglicht wird, ohne daß gleichzeitig eine substantielle Zerstörung des Ringsystems der Steroidverbindung eintritt.

Eine Reihe dieser Vorschläge macht sich das bekannte Prinzip zunutze, durch Zugabe von Inhibitoren zur Nährlösung das den Ring öffnende Enzymsystem der Mikroorganismen zu hemmen. Ein hierzu geeigneter bekannter Inhibitor ist das α,α'-Dipyridyl. Seine Mitverwendung zum selektiven Seitenkettenabbau an Sterinverbindungen in C-17 ist beispielsweise beschrieben in Chemical Abstracts, Band 87 (1977), S. 464, linke Spalte. Nach diesem Vorschlag gelingt es, mit Nocardia corallina IFO 3338 in Gegenwart von α,α'-Dipyridyl eine beschränkte Menge 3-Oxo-pregna-1,4-dien-20-carbonsäure (Δ-1,4 BNC) zu gewinnen.

Ein entsprechender weiterer Vorschlag - siehe Chemical Abstracts, Band 86 (1977), S. 304, rechte Spalte - sieht vor, ß-Sitosterin mit dem Stamm Nocardia sp. M 29 in Gegenwart von α,α'-Dipyridyl abzubauen. Als Reaktionsprodukt fallen in geringer Ausbeute modifizierte Sterinverbindungen an, bei denen der Substituent in C-17 vollständig abgebaut ist und stattdessen an dieser Stelle eine Oxogruppe vorliegt (4-en-3,17-Dionverbindung bzw. 1,4-en-3,17-Dionverbindung). Die Zugabe von lipophilen organischen Adsorbentien soll die Bildung der 1,4-Dien-3,17-dionverbindung fördern. Außerdem wird in mäßigen Ausbeuten daneben ein Gemisch mehrerer partieller Abbauprodukte erhalten, in denen Restbestände des Seitenkettensubstituenten in C-17 noch erhalten sind.

Andere Vorschläge versuchen durch Mutation von sterinabbauenden Stämmen geeignete Mutanten zu entwickeln, mit denen partielle Abbauleistungen an Sterinverbindungen und insbesondere solchen natürlichen Ursprungs erzielt werden können. Ein entsprechender Vorschlag findet sich in Biochimica et Biophysica Acta, Band 531, 1978, Elsevier, Amsterdam, NL, Verf.: M.G. Wovcha et al "Bioconversion of sitosterol to useful steroidal intermediates by mutants of mycobacterium fortuitum", S. 308 - 320. Im einzelnen wird hier die Umwandlung von β-Sitosterin in einer Reihe von Abbauprodukten beschrieben, die durchweg statt des ursprünglich vorliegenden Seitenkettensubstituenten in C-17 eine Oxogruppe aufweisen. Unter anderem wird hier die Herstellung der 4-en-3,17-Dion-Verbindung beschrieben.

Ziel der vorliegenden Erfindung ist demgegenüber die Schaffung eines sicher reproduzierbaren Verfahrens zur Gewinnung von Mikroorganismen-Blockmutanten, die zur technischen Gewinnung von 17-C-Steroid-α-propionsäureverbindungen, insbesondere zur Herstellung von 3-Oxo-pregna-4-en-20-carbon-säure (Δ-4 BNC) und/oder 3-Oxo-pregna-1,4-dien-20-carbonsäure (Δ-1,4 BNC) aus Sterinverbindungen tierischen oder pflanzlichen Ursprungs geeignet sind. Diese Mikroorganismen-Blockmutanten sollen einen wenigstens weitgehend selektiven Seitenkettenabbau ermöglichen, auch ohne daß der Zusatz von das Mikroorganismen-Wachstum und/oder den Steroidringabbau hemmenden Inhibitoren erforderlich ist. Es leuchtet ein, daß für ein technisches Verfahren diese Arbeitsweise in Abwesenheit von Inhibitoren wünschenswert oder sogar erforderlich ist, weil nur auf diese Weise mit hinreichenden Raum-/Zeit-Durchsätzen gearbeitet werden kann.

Gegenstand des EP 004 913, im folgenden als Patent I bezeichnet, ist unter anderem ein Verfahren zur Herstellung von Mikroorganismen-Blockmutanten die zur technischen Gewinnung von 17-C-Steroid-α-propionsäureverbindungen, insbesondere zur Herstellung von 3-Oxo-pregna-4-en-20-carbonsüure (Δ-4 BNC) und/oder 3-Oxo-pregna-1,4-dien-20-carbonsäure (Δ-1,4 BNC), aus 17-C-Seitenketten-Steroidsubstraten, z.B. aus Sterinverbindungen tierischen oder pflanzlichen Ursprungs, durch wenigstens weitgehend selektiven Seitenkettenabbau auch in Abwesenheit von den Steroidringabbau und/oder das Wachstum hemmenden Inhibitoren unter aeroben Bedingungen befähigt sind. Das Verfahren des Patents I ist dadurch gekennzeichnet, daß man

1. einen Mikroorganismen-Wildstamm isoliert und züchtet, der zum Wachstum auf Sterinverbindungen als alleiniger Kohlenstoffquelle befähigt ist und dabei den 17-C-Seitenkettenabbau einem Ringabbau gegenüber bevorzugt,
2. den Wildstamm einer an sich bekannten Mutantionsbehandlung unterwirft,
3. die Mutantenpopulation auf einem Trennmedium züchtet, auf dem die 17-C-Steroid-α-propionsäureverbindungen liefernden Mutanten praktisch nicht wachsen, während unerwünschten begleitenden Mutantenstämme wachsen und hierdurch bzw. während ihres Wachstums abgetötet werden und
4. den verbliebenen Rest der Mutantenstämme auf 17-C-Seitenkettensterinen züchtet und dabei die Stämme mit optimaler Produktion der 17-C-Steroid-α-propionsäureverbindungen isoliert.

Δ-4 BNC und Δ-1,4 BNC sind wertvolle Zwischenprodukte zur Herstellung von Steroidverbindungen der Progesteron-Serie. Ihre Strukturformeln sind die folgenden:

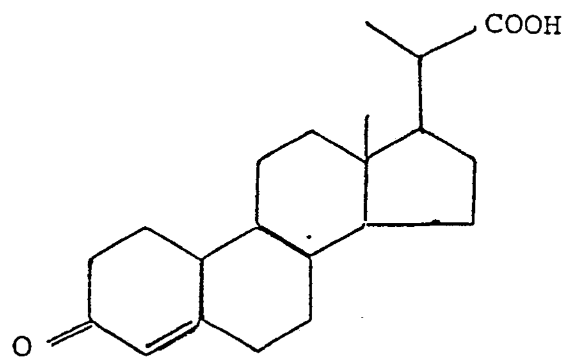

(Δ-4 BNC)

3-Oxo-pregna-4-en-20-carbonsäure

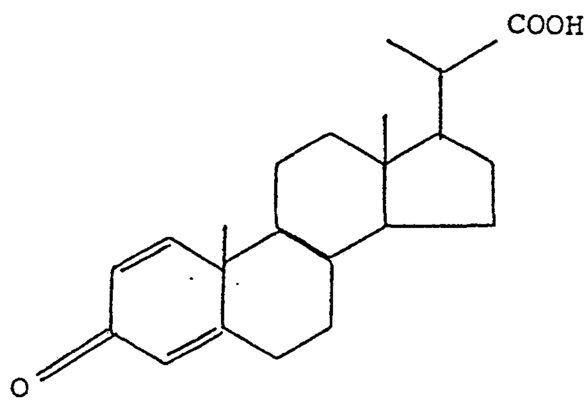

(Δ-1,4 BNC)

3-Oxo-pregna-1,4-dien-20-carbonsäure.

Bevorzugt werden im Verfahren des Patents I in der Verfahrensstufe 1 Wildstämme ausgewählt und unter aeroben Bedingungen gezüchtet, die beim Wachstum auf Sterinverbindungen mit gesättigten oder ungesättigten Alkylresten in 17-C- mit 8 bis 10 C-Atomen eine selektive Abbauleistung - gemessen unter Standardbedingungen wie im allgemeinen Beschreibungsteil des Hauptpatents definiert - gemäß der allgemeinen Formel

$$I = a \cdot 10^b$$

ergeben, worin a der Wachstumsfaktor und b der Selektivitätsfaktor des Wildstammes sind und der Selektivitätsindex den Zahlenwert von wenigstens 10, vorzugsweise von wenigstens 100 und insbesondere den Wert von wenigstens $10^5$ beträgt. Besonders bevorzugt wird es dabei, Wildstämme auszuwählen und in der anschließenden Mutation einzusetzen, deren Selektivitätsfaktor b wenigstens 1, vorzugsweise wenigstens 2, und deren Wachstumsfaktor a vorzugsweise ebenfalls wenigstens 1 beträgt, wobei es weiterhin bevorzugt sein kann, die Wildstämme zunächst nach ihrem Selektivitätsfaktor b - bei möglichst hohen Zahlenwerten für b - und erst dann nach ihrem Wachstumsfaktor a zu berücksichtigen.

Die Bestimmung dieses Selektivitätsfaktors b erfolgt dabei nach den Angaben des Patents I durch aerobe Züchtung der Wildstämme in Parallelversuchen auf radioaktiv markierten Sterinverbindungen, z.B. 4-14 C- und 26-14 C-Cholesterin oder den entsprechend radioaktiv markierten Sitosterinverbindungen. Dabei wird zweckmäßigerweise in sogenannten Warburg-Gefäßen gearbeitet. Das bei der jeweiligen Substratspaltung entstehende radioaktive $CO_2$ wird in einer Base aufgefangen und im Scintillations-Counter gemessen. Der Selektivitätsfaktor b ergibt sich als das dimensionslose Verhältnis der so gemessenen Radioaktivität

$$b = \frac{{}^{14}CO_2\text{-Sterinverbindung von (26-}{}^{14}\text{C)-Sterinverbindung}}{{}^{14}CO_2\text{ aus Ringsystem von (4-}{}^{14}\text{C)-Sterinverbindung}}$$

Diese Auswahl der Wildstämme in der Lehre des Hauptpatents unter anderem nach ihrem Selektivitätsfaktor b fordert damit das doch recht aufwendige Arbeiten mit jeweils zwei radioaktiv markierten Sterinverbindungen in Parallelversuchen.

Gegenstand des Patents I ist weiterhin ein Verfahren zur Herstellung von 17-C-Steroid-α-propionsäureverbindungen, insbesondere Δ-4 BNC und/oder Δ-1,4 BNC, durch mikrobiellen Seitenkettenabbau an 17-C-Seitenketten-Steroidsubstraten unter aeroben Bedingungen, das dadurch gekennzeichnet ist, daß man gewünschtenfalls auch in Abwesenheit von Inhibitoren für den Steroidringabbau und/oder das Wachstum hemmenden Inhibitoren mit Defektblockmutanten als Mikroorganismen arbeitet, die gemäß dem zuvor geschilderten Herstellungsverfahren gewonnen worden sind.

Gegenstand der vorliegenden Erfindung ist demgegenüber eine weitere Ausgestaltung dieser verschiedenen Aspekte der Lehre des Patents I. Überraschenderweise wurde gefunden, daß sich als Mikroorganismen-Wildstämme für die anschließende Mutation, Trennung und schließlich Züchtung der Mutantenpopulation und letztendlich damit für das Verfahren zur Herstellung von 17-C-Steroid-α-propionsäureverbindungen aus 17-C-Seitenketten-Steroidsubstraten besonders solche Mikroorganismen-Wildstämme eignen, die beim Wachstum auf Sterinverbindungen der genannten Art in Gegenwart von Inhibitoren für den enzymatischen Ringabbau der Sterinverbindungen wenigstens anteilsweise 17-C-Steroid-α-propion-säureverbindungen liefern.

Die vorliegende Weiterentwicklung unterscheidet sich damit von dem eingangs geschilderten Verfahren des Patents I zur Herstellung der gewünschten Mikroorganismen-Defektmutanten in der Stufe 1, d.h. in der Stufe der Auswahl des jeweiligen Mikroorganismen-Wildstamms. Während nach der Lehre des Patents I geeignete Mikroorganismenstämme dadurch identifiziert werden, daß ihre Enzymaktivität einerseits für den Seitenkettenabbau und andererseits für den Ringabbau bestimmt und miteinander verglichen werden, wird als Kriterium für die Eignung des Wildstamms gemäß der vorliegenden Weiterentwicklung die Fähigkeit des jeweiligen Wildstamms zugrundegelegt, Sterinverbindungen mit bestimmter Seitenkettensubstitution in 17-C- in Gegenwart von Inhibitoren für den Sterinringabbau zu liefern.

Inhibitoren für den enzymatischen Ringabbau bei Züchtung von Mikroorganismen auf Sterinverbindungen sind im Stand der Technik ausführlich geschildert, vergleiche hierzu beispielsweise Ch.K.A. Martin "Microbial Cleavage of Sterol Side Chains", Adv. Appl. Microbiol. 22, 29-58 (1977), insbesondere das Unterkapitel IV, B, Seiten 44-50 a.a.O. sowie Nagasawa et al.: Agr. Biol. Chem. 34, 838-844 (1970).

Daß gewisse Mikroorganismenwildstämme in Gegenwart solcher Inhibitoren gelegentlich in der Lage sein können, aus natürlichen tierischen oder pflanzlichen Sterinverbindungen, wie Cholesterin oder Sitosterin Δ-1,4 BNC zu bilden, ist kürzlich berichtet worden, siehe hierzu K. Arima et al. "Microbial Production of 3-Oxobisnorchola-1,4-dien-22-oic Acid" in Agric. Biol. Chem. 42 (2), 411-416 (1978). Das erfindungsgemäße Verfahren setzt solche Mikroorganismenwildstämme als Ausgangsmaterial zur Gewinnung verbesserter und wirkungsvollerer Defektmutanten ein. Bevorzugt werden dabei bestimmt ausgewählte Wildstämme dieser Art als Basis für die Gewinnung der erfindungsgemäßen Defektmutanten verwendet.

Der erfindungsgemäßen Abwandlung des bzw. der Verfahren des Patents I zur Identifizierung geeigneter Mikroorganismen-Wildstämme für die anschließende Mutationsbehandlung liegen zwei Feststellungen zugrunde: Bei der Suche nach Sterinverbindungen der genannten Art verzehrenden Mikroorganismen können mit überraschend hoher Trefferquote solche Stämme isoliert werden, die in Gegenwart von Inhibitoren für den enzymatischen Ringabbau der Sterinverbindungen 17-C-Steroid-α-propionsäureverbindungen und insbesondere Δ-4 BNC und/oder Δ-1,4 BNC liefern. Tatsächlich sind solche Stämme auch schon beschrieben und durch Hinterlegung erfaßt wie zuvor erwähnt. Weiterhin wurde festgestellt, daß Wildstämme dieser Art besonders geeignete Ausgangsmaterialien für die nachfolgende Mutation und Selektion im Sinne der Lehre des Hauptpatents sind, um dabei letztlich Defektbutanten zu liefern, die zur Gewinnung der 17-C-Steroid-α-propionsäureverbindungen durch mikrobiellen Seitenkettenabbau an 17-C-Seitenketten-Steroidsubstraten in einem technischen Verfahren und in guten Ausbeuten geeignet sind. Defektmutanten der erfindungsgemäßen Art sind insbesondere auch zur Inhibitor-freien Herstellung von Δ-4 BNC und/oder Δ-1,4 BNC geeignet.

Als besonders geeignet haben sich solche Wildstämme der genannten Art für die anschließende Mutation und Selektion erwiesen, die beim Wachstum auf Sterinverbindungen mit gesättigten oder ungesättigten Alkylresten in 17-C-Stellung mit vorzugsweise 8 bis 10 C-Atomen unter Standardbedingungen eine Ausbeute an 17-C-Steroid-α-propionsäureverbindun-gen von wenigstens 5 Gew.-%, vorzugsweise von wenigstens 10 Gew.-% - jeweils bezogen auf die im Standardversuch eingesetzte(n) Sterinverbindung(en) - liefern. Die Bedingungen dieses Standardversuchs werden dabei im folgenden im Zusammenhang mit der Bestimmung des Selektivitätsfaktors p beschrieben.

Die in der erfindungsgemäßen Selektionsstufe ermittelten bevorzugten Wildstämme im Sinne der Erfindung bilden bei ihrer Züchtung auf Sterinen als einzigem Kohlenstofflieferanten in einem üblichen Nährmedium bei Zusatz von Inhibitoren für den enzymatischen Ringabbau am Sterin in der Regel Δ-4 BNC und/oder Δ-1,4 BNC. Die Selektion der Mikroorganismen im Rahmen der vorliegenden Erfindung kann dementsprechend in einer bevorzugten Ausführungsform auf das Bildungsvermögen der Mikroorganismen-Wildstämme - d.h. Ausbeute - von Δ-4 BNC und/oder Δ-1,4 BNC unter Standardbedingungen ausgerichtet werden. Die Bestimmung dieser Fähigkeit und damit des Selektivitätsfaktors p des Wildstamm-Wachstums erfolgt dabei unter den folgenden Standardbedingungen:

**Ausbeute bzw. Selektivitätsfaktor p des Wildstamms**

Der jeweilige Mikroorganismenstamm wird in 500 ml Erlenmeyerkolben (100 ml Nährlösung der im folgenden beschriebenen Zusammensetzung) bei 30°C auf der Schüttelmaschine (Schüttelfrequenz: 150 U/min) inkubiert. Hier und in allen weiteren Schritten der Mikroorganismen-Züchtung wird unter aeroben Bedingungen gearbeitet.

Das Nährmedium hat die folgende Zusammensetzung (jeweils in Gew.-%):

0,20 % $K_2HPO_4$
0,05 % $NaH_2PO_4$
0,80 % Pepton
0,90 % Hefeextrakt
0,30 % Glucose
pH 7,2

Nach 48 Stunden Kulturdauer bei 30°C erfolgt die Zugabe von 0,1 % Tween 80 (Polyoxyäthylensorbitanmonooleat) und 0,1 % 17-C-Seitenketten-Sterinverbindungen, z.B. Cholesterin oder Sitosterin. Nach weiteren 4 Stunden erfolgt die Zugabe des Inhibitors in einer Konzentration von $10^{-3}$ Mol/l (M).

Zum Zeitpunkt der Inhibitorzugabe wird der $p_H$-Wert des Mediums auf 8,0 justiert. Nach weiterer Inkubation von 62 Stunden wird die Kultur geerntet und der Gehalt an Δ-4 BNC und/oder Δ-1,4 BNC nach üblichen Methoden in Gew.-%, bezogen auf eingesetzte Sterinverbindung, bestimmt.

Der Selektivitätsfaktor p errechnet sich dabei aus der prozentualen BNC-Ausbeute gemäß

$$p = \frac{\text{\% BNC-Ausbeute}}{10}$$

Innerhalb der Gruppe der in Gegenwart von Inhibitoren BNC bildenden Mikroorganismenstämme werden für die Erfindung in der Regel die Stämme um so geeigneter, je höher die BNC-Ausbeute und damit je größer der Selektivitätsfaktor p im Einzelfall ist. Als unterer Grenzwert für geeignete Wildstämme ist im allgemeinen ein Selektivitätsfaktor p von wenigstens 0,5 anzusehen, wobei jedoch Stämmen mit Selektivitätsfaktoren p von wenigstens 1 oder wenigstens 1,5 der Vorzug gegeben wird. Als besonders geeignet haben sich Stämme mit Selektivitätsfaktoren p von 2 oder mehr erwiesen, wobei nach den besten bisher bekannten Ergebnissen Selektivitätsfaktoren in der Größenordnung von 4 ermittelt worden sind.

Als Inhibitoren zur Hemmung des enzymatischen Abbaus des Sterinringsystems eignen sich aus der hierfür bekannten Gruppe von Verbindungen, beispielsweise α,α'-Dipyridyl, o-Phenanthrolin, 8-Oxochinolin und ganz allgemein Mittel, die unter Chelatbildung Komplexsalze mit Schwermetallen bilden, wie 1-Nitroso-2-naphthol, Salicylaldoxim, Nitrosophenylhydroxylamin, 1-Nitroso-2-naphthol-3,6-disulfonsäure, Tetrahydroxyanthrachinon, und dergleichen. Bevorzugte Inhibitoren für die Durchführung des Standardtestes zur Bestimmung des Selektivitätsfaktors p sind α,α'-Dipyridyl und o-Phenanthrolin, wobei es für die Ausbeutebestimmung nach dem Standardtest ausreichend ist, wenn die geforderte Mindestausbeute mit einem dieser beiden Inhibitoren unter sonst gleichen Züchtungsbedingungen erzielt wird.

Unabhängig von der Bestimmung des Selektivitätsfaktors p erfolgt die bevorzugte Auswahl geeigneter Wildstämme im Rahmen der Erfindung unter zusätzlicher getrennter Bestimmung der Wachstumsgeschwindigkeit der jeweiligen Wildstämme. Hier gelten die Angaben des Patents I zur Ermittlung des entsprechenden Wachstumsfaktors a.

**Wachstumsfaktor a**

Der jeweilige Mikroorganismenstamm wird in 500 ml Erlenmeyerkolben (100 ml Nährlösung der folgenden beschriebenen Zusammensetzung) bei 30°C unter aeroben Bedingungen auf der Schüttelmaschine (Schüttelfrequenz: 150 U/min.) inkubiert.

Das Nährmedium hat die folgende Zusammensetzung (pro Liter):

1,0 g $KH_2PO_4$
0,5 g $MgSO_4 \cdot 7\,H_2O$
0,1 g NaCl
0,1 g $CaCl_2 \cdot 2\,H_2O$
5 g $(NH_4)_2SO_4$
1,0 g Tween 80 (Polyoxyäthylensorbitanmonooleat)
2,0 g der 17-C-Seitenkettensterinverbindung, z.B. Cholesterin oder Sitosterin
0,5 g Hefeextrakt
0,01 g 1-Histidin
0,02 g dl-Methionin
0,02 g dl-Tryptophan
2 µg Biotin
400 µg Calciumpantothenat
2 µg Folsäure
2000 µg Inosit
400 µg Niacin
200 µg p-Aminobenzoesäure
400 µg Pyridoxinhydrochlorid
200 µg Riboflavin
400 µg Thiaminhydrochlorid
Spurenelementlösung
pH-Wert des Nährmediums 6,8.

Zur Messung des Wachstums (Zelldichte) werden in zeitlichen Abständen den Schüttelkulturen aliquote Mengen Zellsuspension entnommen und die jeweilige Extinktion (E') im Zeiss-Photometer (Typ PL 4) bei einer Wellenlänge von 620 nm (Schichtdicke d = 1 cm) gegen destilliertes Wasser gemessen. Die Ausgangsextinktion $E_0$ beträgt vor Beginn des Wachstums beim Zeitpunkt $t_0$ etwa 0,775. Die Suspensionen werden jeweils soweit verdünnt, daß im Bereich E' = 0,7 gemessen werden kann.

Der Wachstumsfaktor a ergibt sich als die maximale optische Dichte der Zellsuspension am Ende der logarithmischen Wachstumsphase (Zeitpunkt $t_1$ Extinktion $E_1$), die unter den gegebenen Kulturbedingungen nach spätestens 5 Tagen erreicht wird, d.h. $a = \Delta E = E_1 - E_0$.

Erfindungsgemäß sind für die nachfolgende Mutation und Selektion besonders solche Mikroorganismen-Wildstämme geeignet, die unter Berücksichtigung der zuvor geschilderten Faktoren a und p eine selektive Abbauleistung gemäß der allgemeinen Formel

$I = a \cdot 10^p$

(I = Selektivitätsindex) mit einem Zahlenwert von wenigstens 1, vorzugsweise von wenigstens 2 und insbesondere von wenigstens 20 liefern. Auch noch beträchtlich höhere Zahlenwerte können in Betracht kommen, z.B. Werte von $I \geqq 1 \cdot 10^3$ oder gar $\geqq 5 \cdot 10^3$. Im Rahmen der Erfindung werden dabei Wildstämme mit Wachstumsfaktoren a von wenigstens 0,2, vorzugsweise von wenigstens 1, insbesondere von 4 oder mehr, besonders bevorzugt.

Auch im Rahmen der vorliegenden Weiterentwicklung kann es für die Auswahl der für die weiteren Stufen des erfindungsgemäßen Verfahrens am besten geeignete Wildstämme zweckmäßig sein, die Faktoren a und p gegeneinander abzuwägen. So kann es im Rahmen der Erfindung zweckmäßig sein, Wildstämme mit einem besonders hohen Selektivitätsfaktor p den Vorzug zu geben und bei der Wahl zwischen verschiedenen isolierten Wildstämmen zunächst nach diesem Selektivitätsfaktor p bei möglichst hohen Zahlenwerten für p auszuwählen. Umgekehrt kann natürlich auch ein besonders gutes Wachstum - ausgedrückt durch einen besonders hohen Zahlenwert für den Wachstumsfaktor a - Anlaß sein, für die anschließende Mutation und darauffolgende Selektion der Mutantenpopulation einem solchen Wildstamm den Vorzug zu geben.

In Gegenwart von Inhibitoren Δ-4 BNC und/oder Δ-1,4 BNC liefernde Wildstämme finden sich in großer Breite der Mikroorganismen-Klassifikation. Geeignet sind beispielsweise solche von Achromobacter, Arthrobacter, Bacillus, Brevibacterium, Corynebacterium, Flavobacterium, Microbacterium, Mycobacterium, Nocardia, Protaminobacterium, Serratia oder Streptomyces. Wie erwähnt gibt es auch in der Literatur bereits Hinweise auf Stämme, die bei der Züchtung auf 17-C-Seitenketten-Sterinen in Gegenwart von Inhibitoren, wie α,α'-Dipyridyl und o-Phenanthrolin, Δ-1,4 BNC mit unterschiedlichen Ausbeuten liefern, vergleiche K. Arima et al., a.a.O.

Nachdem die Auswahl des geeigneten Wildstammes erfolgt ist, kann dann das weitere Mutations- und Selektionsverfahren im Sinne der Angaben des Patents I erfolgen. Im einzelnen gilt hierfür:

**Mutationsbehandlung (Stufe 2):**

Die Mutation des ausgewählten Wildstammes erfolgt in an sich bekannter Weise. Sie kann beispielsweise durch energiereiche Bestrahlung etwa mit UV oder Röntgenstrahlung erfolgen. Geeignet ist insbesondere aber auch die Behandlung der Zellen mit mutagenen Agentien. Geeignete mutagene Agentien sind beispielsweise Nitrosoguanidinverbindungen, wie 1-Methyl-3-nitro-1-nitrosoguanidin oder Äthylmethansulfonat. Im einzelnen kann hier auf die allgemeinen Angaben des Standes der Technik verwiesen werden, siehe hierzu beispielsweise US-PS 4 029 549, Spalte 2, Zeilen 57 ff mit den dort enthaltenen Verweisen.

Grundsätzlich gilt auch für die Erfindung, daß das Ergebnis der Wildstamm-Mutation insoweit unbestimmt ist, als die Eigenschaften der erhaltenen Defektmutanten im einzelnen nicht vorausbestimmbar sind.

Trotzdem lassen sich Prinzipien für eine optimale Mutationsauslösung festlegen, da die Gesetze der Statistik auf eine Mikroorganismenpopulation anwendbar sind. Aus der Literatur sind Verfahren bekannt, die optimalen Bedingungen für die Induktion von Mutationen zu bestimmen (s.a. E.A. Adelberg, M. Mandel, GCC Chen (1965) "Optimal Conditions for Mutagenisis by N-methyl-N'-nitro-N-nitrosoguandine in Escherichia coli", Biochem. Biophys. Res. Commun. 18, 788-795).

Im allgemeinen wird zur Erhöhung der Häufigkeit von Mutationen in Mikroorganismenpopulationen die Konzentration und die Einwirkzeit der mutagenen Agentien so gewählt werden, daß bereits ein Teil der Mikroorganismenpopulation letal geschädigt ist. Dabei steigt in dem überlebenden Teil der Population die Häufigkeit der verschiedensten Mutationen mehr oder weniger stark an.

Im Rahmen des erfindungsgemäßen Verfahrens werden die Bedingungen von Konzentration und Einwirkzeit des mutagenen Agens so gewählt, daß die Ausgangspopulation durch die Behandlung zu 10-99,999 % inaktiviert wird. Vorzugsweise wird eine Abtötungsrate von 90-99,99 % gewählt.

An die Verfahrensstufe 2 der Mutation schließen sich die Verfahrensstufen 3 und 4 an.

**Zu Stufe 3**

Zur nachfolgenden Auslese der erfindungsgemäß bestimmt gewünschten Mutanten aus der großen Population der Mikroorganismen nach der Mutationsbehandlung werden erfindungsgemäß Kulturbedingungen gewählt, unter denen die abgeänderten spezifischen Eigenschaften der entstandenen Mutantenstämme zum Selektionsvorteil werden. Die Anreicherung der erwünschten Defektmutanten erfolgt erfindungsgemäß häufig unter Bedingungen, unter denen die spezielle Mutante nicht oder praktisch nicht wächst, während

unerwünschte Begleitorganismen wachsen und durch ihr Wachstum oder bei ihrem Wachstum abgetötet werden. Auf diese Weise gelingt es, diejenigen Defektmutantenstämme zu isolieren, deren ringabbauende Enzyme blockiert sind, jedoch nach wie vor zum Abbau der 17-C-Seitenkette an der Sterinverbindung befähigt sind, wobei durch weitere Maßnahmen insbesondere dieser Stufe 3 sichergestellt wird, daß gerade solche Defektmutanten isoliert werden können, die beim Seitenkettenabbau bevorzugt den α-Propionsäurerest in 17-C-Stellung ausbilden.

Hierzu wird in der Verfahrensstufe 3 die Mutantenpopulation auf einem "Trennmedium" gezüchtet, das letztlich als Anreicherungsmedium für die gewünschten Mutantenstämme dient. Als Mutanten-Trennmedium kann ein wäßriges Nährmedium eingesetzt werden, das als Kohlenstoffquelle eine Steroidverbindung mit einer 17-C-Seitenkette mit nur beschränkter C-Zahl oder auch keine Seitenkette in 17-C-Stellung enthält. Geeignet sind als Kohlenstoffquelle in diesem Mutanten-Trennmedium neben den in 17-C-Stellung nicht substituierten Steroidverbindungen insbesondere solche, die Seitenketten mit bis zu 5 C-Atomen enthalten.

Bevorzugt ist es allerdings, als Kohlenstoffquelle in diesem Mutantentrenn- bzw. Anreicherungsmedium eine Steroidverbindung mit 3 C-Atomen in der 17-C-Seitenkette einzusetzen, wobei zweckmäßigerweise hier eine 17-C-Steroid-α-propionsäureverbindung oder auch eine Mehrzahl solcher gewünschter 17-C-Steroid-α-propionsäureverbindungen als einzige Kohlenstoffquelle verwendet wird.

Es kann dabei weiterhin bevorzugt sein, in diesem Mutantentrennmedium als Kohlenstoffquelle diejenigen 17-C-Steroid-α-propionsäureverbindungen einzusetzen, deren Herstellung mittels der erfindungsgemäß gezüchteten Defektmutanten letztendlich angestrebt wird. Soll also im Rahmen der Erfindung aus Sterinen pflanzlichen oder tierischen Ursprungs beispielsweise Δ-4 BNC und/oder Δ-1,4 BNC letztendlich als Verfahrensprodukt gewonnen werden, so kann es zweckmäßig sein, in dem Mutantentrenn- bzw. Anreicherungsmedium dieser Stufe 3 als einzige Kohlenstoffquelle Δ-4 BNC und/oder Δ-1,4 BNC einzusetzen.

Im übrigen wird hier mit konventionellen Nährlösungen unter aeroben Bedingungen gearbeitet.

Diejenigen mutierten Mikroorganismen, die aufgrund der Mutationsbehandlung ihre Fähigkeit verloren haben, auf den jetzt vorliegenden Kohlenstoffquellen zu wachsen, können sich beim Bebrüten des Mutantentrennmediums nicht vermehren. Sie wachsen also nicht oder nicht wesentlich. Ein anderer Teil der Mutantenpopulation, der entweder bei der Mutationsbehandlung der Stufe 2 nicht hinreichend geschädigt worden ist oder andere Defekte erlitten hat, ist befähigt, auf der Kohlenstoffquelle des Mutantentrennmediums zu wachsen. Bei der Bebrütung tritt hier also eine Vermehrung ein.

Die Erfindung macht sich dieses unterschiedliche Verhalten derart zunutze, daß die Bedingungen im Mutantentrennmedium so gewählt werden, daß die wachsenden Stämme aufgrund ihres Wachstums oder während ihres Wachstums abgetötet werden, ohne daß dabei die nicht wachsenden Mutantenstämme geschädigt werden.

Möglich ist eine solche Trennung beispielsweise durch Zusatz von Antibiotika, beispielsweise durch Zusatz von Penicillinverbindungen. Die Zugabe von Penicillinverbindungen führt zur Abtötung des Anteils der wachsenden Mikroorganismenstämme, während die nicht wachsenden Stämme ungeschädigt bleiben.

Aus den verbliebenen ungeschädigten Blockmutanten können dann z.B. mittels der an sich bekannten Lederbergschen Stempelmethode die erfindungsgemäß angestrebten Defektmutantenstämme isoliert werden. Bezüglich der hier eingesetzten Verfahrenstechnik und zur Penicillinanreicherungsmethode und zur Lederbergschen Stempelmethode wird beispielsweise verwiesen auf J. Amer. Chem. Soc. 70, 4267, (1948) Davis, B.D. (Penicillin-Anreicherungsmethode), J. Bact. 63, 399 (1952), Lederberg, J., Lederberg, E.M. (Lederbergsche Stempelmethode).

Eine andere erfindungsgemäß geeignete Methode ist die Abtötung der ungeeigneten Mutanten durch Verwendung von Nährmedien, die radioaktive Komponenten enthalten, welche letztlich die wachsenden Zellen insbesondere unter Einbau in die Zellstruktur schädigen. Geeignet ist hier beispielsweise die Abtötung der ungeeigneten Mutanten durch ein Nährmedium, das $^{32}P$ insbesondere in Form des Salzes $NaH_2{}^{32}PO_4$ enthält, zu dieser Inaktivierungstechnik siehe Fuerst, C.R., Stent, G.S.: Inactivation of Bacteria by Decay of Incorporated Radioactive Phosphorus, J. Gen. Physiol. 40, 73-90 (1956).

**Zu Stufe 4**

Die so isolierten und selektierten Defektmutanten können jetzt auf einem üblichen Nährmedium angezüchtet werden und dann gewünschtenfalls einer weiteren Selektion unterworfen werden. Die Selektion kann hier beispielsweise nach dem angestrebten Ergebnis der Mikroorganismen-Stämme auf dem beabsichtigten Einsatzmaterial, beispielsweise also auf natürlichen oder pflanzlichen Sterinverbindungen erfolgen, wobei hier insbesondere die chemische Natur der Stoffwechselprodukte des Mikroorganismen-Wachstums und die Wachstumsfreudigkeit der Stämme bestimmend sein können. Naturgemäß wird man Defektmutanten mit optimaler Produktivität des letztlich gewünschten Verfahrensprodukts bevorzugen. Diese Stämme können dann im technischen Verfahren eingesetzt werden. Anzüchtung und Selektion können hier einfach oder mehrfach wiederholt werden.

Im Rahmen des erfindungsgemäßen Verfahrens ist es aber auch möglich, die Folge der Verfahrensstufen der Mutation gemäß Stufe 2 und der anschließenden Mutantentrennung gemäß Stufe 3 einfach oder mehrfach zu wiederholen, sofern eine noch stärkere Einwirkung der mutagenen Agentien auf die defekten

Mutantenstämme zur Erzielung letztendlich optimaler Produktionsergebnisse wünschenswert erscheint. Abschließend wird dann in der Regel gemäß Stufe 4 aufgearbeitet.

Erfindungsgemäß kann es bevorzugt sein, die besonders erwünschten Defektmutantenstämme durch ihre Transformationsleistung in einem Standardtest zu ermitteln. Bestimmt wird hierbei die Leistungsfähigkeit des Stammes bezüglich der Bildung von Δ-4- und/oder Δ-1,4 BNC. Für die Ausbeutebestimmung nach diesem Standardtest gelten dabei die folgenden Bedingungen:

**BNC-Ausbeute nach Standardtest**

Der zu prüfende Defektmutantenstamm wird im 500 ml Erlenmeyerkolben mit 100 ml Nährlösung folgender Zusammensetzung gezüchtet: 0,8 % Pepton, 0,9 % Hefeextrakt, 0,3 % Glukose, 0,06 % Tween 80 (Polyoxyäthylensorbitanmonooleat), 0,06 % Sterinverbindung (Cholesterin oder Sitosterin), $p_H$ 7,2. Die Kultur wird auf der Schüttelmaschine (Schüttelfrequenz 150 UpM) bei 30°C für 62 Stunden vorgezüchtet. Anschließend werden 0,1 % BRIJ 35 (Polyoxyäthylenmonolauryläther) und 0,1 % der zuvor gewählten Sterinverbindung (Cholesterin oder Sitosterin) zugegeben und für weitere 120 Stunden bebrütet. Nach Abbruch der Kulturen werden Proben genommen und diese extrahiert und dünnschichtchromatographisch analysiert. Die prozentuale Ausbeute an Δ-4-und/oder Δ-1,4 BNC wird bestimmt. Sämtliche prozentangaben sind Gewichts-%. Die Ausbeute bezieht sich auf die im Standardversuch eingesetzte Gewichtsmenge der zu transformierenden Sterinverbindung.

Die im Sinne der Erfindung bevorzugten Defektmutantenstämme liefern unter diesen Standardbedingungen mit Cholesterin oder Sitosterin als Steroidsubstrat eine Mindestausbeute von 15 Gew.-%, bevorzugt Ausbeuten von 30 Gew.-% und mehr. Bei der Verwendung von Cholesterin als Steroidsubstrat kann die bevorzugte Mindestausbeute im Sinne dieses Standardtestes 50 Gew.-% oder mehr ausmachen, wobei besonders wirkungsvolle Stämme BNC-Ausbeuten im Bereich von 70 bis 80 Gew.-% liefern. Bei der Verwendung von Sitosterin als Steroidsubstrat kann die Ausbeute etwas niedriger liegen, aber auch hier Werte im Bereich von 40 bis 50 Gew.-% erreichen.

Auch die Lehre der vorliegenden Erfindung erfaßt weiterhin das Verfahren zur Herstellung von 17-C-Steroid-α-propionsäureverbindungen und insbesondere die Herstellung von 3-Oxo-pregna-4-en-20-carbonsäure und/oder 3-Oxo-pregna-1,4-dien-20-carbonsäure mittels der so gewonnenen Defektblockmutanten aus Sterinverbindungen mit Seitenketten in 17-C-Stellung insbesondere tierischen oder pflanzlichen Ursprungs.

Neben oder anstelle der Verwendung von Steroidsubstraten natürlichen Ursprungs wie Cholesterin, Sitosterin usw. können auch davon abgeleitete Verbindungen wie Cholestenon, Sitostenon, Stigmastenon und dergleichen als Ausgangsmaterial Verwendung finden.

Die selektive Umwandlung des als Ausgangsmaterial gewählten Steroidsubstrats, insbesondere also einer natürlichen Sterinverbindung, kann nach Schaffung und Auswahl der Defektmutante gemäß dem zuvor beschriebenen Verfahren in an sich bekannter Weise erfolgen. So kann also beispielsweise die als Einsatzmaterial gewählte Steroidverbindung in der Kultur während der Inkubationsperiode zugesetzt werden oder sie kann dem Nährmedium vor der Inokulierung der Defektmutanten beigegeben werden. Es kann eine Steroidverbindung oder auch eine Mischung von mehreren Steroidverbindungen eingesetzt werden. Vorzugsweise werden die selektiv abzubauenden Steroidverbindungen in der Kultur in Mengen von etwa 0,1 bis 100 g/l verwendet. Die optimale Konzentration der zu transformierenden Sterinverbindung in der Züchtungsstufe ist im allgemeinen Stamm-abhängig und kann durch einfache Vorversuche jeweils ermittelt werden. Im allgemeinen liegt die Konzentration der Sterinverbindung im Medium vorzugsweise nicht über 20 g/l und häufig nicht über 15 g/l, jedoch werden Mengen über 1 g/l bevorzugt.

Es kann dabei bevorzugt sein, das dem Seitenkettenabbau zu unterwerfende Substrat nicht auf einmal dem Reaktionsmedium zuzusetzen, sondern diese Zugabe nach und nach im Verlauf der Reaktion vorzunehmen. Vorzugsweise wird das Ausgangssubstrat in dieser Ausführungsform im wesentlichen kontinuierlich im Reaktionsgemisch im Verlauf der Abbaureaktion zugegeben. Auf diese Weise kann häufig die Ausbeute der gewünschten Abbauprodukte erhöht werden.

Die Kultur wird in einem Nährmedium gezüchtet, das als Kohlenstoffquelle entweder die zu transformierenden Sterine oder aber auch noch zusätzliche metabolisierbare Kohlenstoffquellen sowie die üblicherweise von diesen Mikroorganismen benötigten Nähr- und Wuchsstoffe enthält. Besonders günstig für das Wachstum der Mikroorganismen sind z.B. Paraffin, Glycerin, Carbonsäuren, Stärke, Dextrin, Saccharose, Glucose, Fructose und zuckerhaltige Abfallstoffe. Geeignete Stickstoffquellen sind Ammoniumsalze, Nitrate, Pepton, Maisquellwasser, Sojamehl, Schlempe und Fischmehl. Weiterhin können Fermentationsbeschleuniger, wie Hefeextrakt und Vitamine zugesetzt werden. Das Nährmedium enthält darüberhinaus zweckmäßigerweise anorganische Salze, wie Natrium-, Kalium- oder Ammoniumphosphate sowie Calcium-, Magnesium-, Mangan- oder Eisensalze.

Die Emulgierung der Sterine in dem Nährmedium erfolgt vorzugsweise mittels bekannter Emulgatoren, z.B. mittels Fettsäure-Sorbitanester oder deren Äthylenoxidaddukten, Polyoxyäthylenmonolauryläther oder Fettsäureamidoalkylbetain.

Das verwendete Kulturmedium wird vor Beginn der Bakterienanzucht zweckmäßigerweise durch Erhitzen sterilisiert. Nach dem Abkühlen und Beimpfen des Kulturmediums mit einer geeigneten Vorkultur des

transformierenden Bakterienstammes wird zwischen 25° bis 55°C inkubiert, vorzugsweise bei 27° bis 30°C. Der $p_H$-Wert der Nährlösung liegt zwischen $p_H$ 4 bis 8,5, vorzugsweise bei 7,0 bis 8,0. Die Kultur wird durch Schütteln, Rühren oder Gaseinleiten mit Sauerstoff versorgt und bis zum vollständigen Abbau des Sterins bis zur gewünschten Stufe inkubiert. Der Abbau des Sterins fordert je nach Substratkonzentration und den anderen Fermentationsbedingungen in der Regel 24 bis 160 Stunden.

Das auf diese Weise hergestellte Verfahrensprodukt, das sich üblicherweise in der Fermentationsbrühe anreichert, kann dann in an sich bekannter Weise aus dem Reaktionsgemisch gewonnen werden. So können beispielsweise BNC-Verbindungen durch Extraktion mit organischen Lösungsmitteln, wie Methylisobutylketon, Essigsäureester, n-Hexanol, n-Octanol, Chloroform oder n-Hexan, aus dem Kulturmedium vor oder nach Abtrennen der Zellen isoliert werden.

Beispielsweise gelingt eine Isolierung von BNC-Verbindungen, indem man einen Liter einer Fermentationslösung, die 1 g BNC enthält, mit etwa gleichen Volumina organischer Lösungsmittel, wie mit Methylisobutylketon, Essigsäureester, n-Hexanol, n-Octanol, Chloroform oder n-Hexan im Perforator, Turborührer oder Scheidetrichter extrahiert. In den beiden letztgenannten Fällen muß zur Abtrennung der BNChaltigen organischen Phase aus der Emulsion ein Zentrifugationsschritt angeschlossen werden.

Nach Verdampfen des Lösungsmittels hinterbleibt ein BNC-haltiger Rückstand, der nach Umkristallisation z.B. aus Benzol zur reinem BNC vom Smp. 233 bis 236°C aufgearbeitet werden kann.

Die Lösungsmittelextraktion ist insbesondere im sauren $p_H$-Bereich möglich. Dazu wird beispielsweise mit 50 %iger $H_2SO_4$ etwa auf $p_H$ 2 eingestellt und mit Methylisobutylketon, Essigsäureester, n-Hexanol, n-Octanol, Chloroform oder n-Hexan in der zuvor angegebenen Weise extrahiert. Nach Eindampfen der organischen Phase wird auch hier ein BNC-haltiger Rückstand erhalten, der wiederum nach Umkristallisation zum Reinprodukt mit dem Smp. 233 bis 236°C führt. Die Extraktion kann auch im neutralen Bereich durchgeführt werden.

Alternativ ist auch eine Reinigung durch Anionenaustausch möglich. Hierzu wird die Fermentationslösung zweckmäßigerweise zunächst aufkonzentriert und dann auf einen alkalischen Wert, beispielsweise $p_H$ 12, eingestellt. Nach Zusatz einer beschränkten Menge Methanol und Verrühren im Turborührer wird die Zellmasse mittels einer Durchlaufzentrifuge abgetrennt. Der wäßrig-methanolische Überstand wird über eine Ionenaustauschersäule gepumpt, die mit einem Anionenaustauscherharz, z.B. in Acetatform, gefüllt ist. Die gebildete BNC wird dabei vollständig im Ionenaustauscher gebunden. Durch Elution mit 10 %iger Essigsäure in Methanol gelangt man zu einem Produkt, das zunächst überwiegend BNC enthält, nach Umkristallisation wird auch hier reine BNC vom Smp. 233 bis 236°C erhalten.

Nach einer bevorzugten Ausführungsform gelingt die Isolierung der BNC-Verbindungen aus der Fermenterflüssigkeit ganz einfach durch Ausfällung im sauren Bereich und Abfiltrieren. Hierzu wird die alkalisch eingestellte Fermenterflüssigkeit zunächst filtriert, um das Zellmaterial und andere feste Bestandteile zu entfernen. Anschließend wird angesäuert, dabei fällt BNC in fester filtrierbarer Form aus und kann z.B. durch einfaches Abnutschen gewonnen werden.

Zum Ansäuern ist jede Mineralsäure verwendbar, es können aber auch organische Säuren, z.B. Essigsäure, und sogar gasförmiges $CO_2$ eingesetzt werden. Bei $p_H$ 5 ist die praktisch vollständige Ausfällung erreicht. Zur besseren Filtrierbarkeit kann es günstig sein, die Suspension kurz zu erhitzen.

Der isolierte Feststoff kann bis zu 90 % BNC-Verbindungen enthalten. Die reinen Verbindungen lassen sich durch Umkristallisation daraus gewinnen.

**Spezielle Einzelheiten zur Erfindung**

Mittels der erfindungsgemäßen Wildstamm-Selektionsmethode konnte in vergleichsweise kurzer Zeit eine größere Zahl von Wildstämmen isoliert werden, die grundsätzlich als Ausgangsmaterial für das erfindungsgemäße Mutationsverfahren geeignet sind. Die wichtigsten dieser Wildstämme sind unter den folgenden Hinterlegungsnummern hinterlegt worden: DSM 1418, DSM 1419, DSM 1423, DSM 1424, DSM 1425, DSM 1426, DSM 1427, DSM 1428, DSM 1429, DSM 1430, DSM 1431, DSM 1432. Weitere Einzelheiten sind in der Zusammenstellung am Ende der Beispiele enthalten.

Bei diesen Mikroorganismen handelt es sich um spezielle Bakterienstämme, die wenigstens anteilsweise den coryneformen Bakterien zuzurechnen sein dürften.

Interessanterweise wurde festgestellt, daß auch der bereits in dem Patent I genannte Wildstamm, der unter der Hinterlegungsnummer CBS 660.77 in Baarn, Niederlande, hinterlegt ist, die erfindungsgemäße Selektionsmethodik erfüllt, d.h. in Gegenwart von Inhibitoren beträchtliche Mengen an Δ-4- und/oder Δ-1,4 BNC-Verbindungen liefert. Auch dieser zu der Gruppe der coryneformen Bakterien gehörende Wildstamm ist damit prinzipiell ein auch im Sinne der vorliegenden Lehre geeignetes Ausgangsmaterial für die anschließende Mutation zu den gewünschten Defektmutanten.

Alle hier genannten und hinterlegten Wild- und Defektmutantenstämme fallen in den Rahmen der Erfindung.

**Beispiel 1**

A Gewinnung geeigneter Wildstämme

Nach an sich üblichen Anreichungsmethoden werden aus Erdproben sterinabbauende Mikroorganismen isoliert, die man zunächst in einem Plattentest auf ihre Fähigkeit untersucht, auf β-Sitosterin oder Cholesterin zu wachsen. Stämme mit deutlicher Koloniebildung werden als Reinkulturen angelegt und weiteren Selektionsverfahren unterworfen.

B Bestimmung des Wachstumsfaktors a

Zur Ermittlung des Wachstumsfaktors a werden die isolierten Reinkulturen unter den im allgemeinen Beschreibungsteil angegebenen Bedingungen aerob angezüchtet. Zur Bestimmung der optischen Dichte der Zellsuspensionen werden jeweils in 24-stündigen Abständen Proben genommen und die Messungen nach den Angaben des Standardtests im allgemeinen Beschreibungsteil durchgeführt. Nach Erreichen der jeweils maximalen Zelldichte wird der Versuch abgebrochen.

C Bestimmung des Selektionsfaktors p

Diejenigen Wildstämme, die im Schüttelkolbentest mit β-Sitosterin bzw. Cholesterin das beste Wachstum zeigen, werden anschließend im Inhibitortest auf ihre Fähigkeit zur intermediären BNC-Anhäufung geprüft. Die aerobe Anzucht der Stämme geschieht zu diesem Zweck in 100 ml Nährlösung folgender Zusammensetzung: 0,20 % $K_2HPO_4$, 0,05 % $NaH_2PO_4$, 0,80 % Pepton, 0,90 % Hefeextrakt, 0,30 % Glukose, $p_H$ 7,2. Die Kulturansätze werden 48 Stunden bei 30° C auf der Schüttelmaschine inkubiert, anschließend mit 0,1 % Tween 80 (Polyoxyäthylensorbitanmonooleat) und 0,1 % Cholesterin bzw. β-Sitosterin versetzt und nach weiteren 4 Stunden $10^{-3}$ M α,α'-Dipyridyl zugegeben. Zum Zeitpunkt der Inhibitorzugabe wird der $p_H$-Wert des Mediums auf 8,0 justiert. Die Kulturen werden nach 62 Stunden geerntet und der Gehalt an BNC nach üblicher Methode dünnschichtchromatographisch bestimmt.

D Wildstämme, die der erfindungsgemäßen Definition entsprechen

Die wirkungsvollsten der getesteten Stämme wurden bei der Deutschen Sammlung von Mikroorganismen, Grisebachstraße 8, 3400 Göttingen, Deutschland, hinterlegt. Wie bereits angegeben, entspricht auch der im Patent I genannte Wildstamm CBS 660.77 der hier gegebenen Definition.

| Hinterlegungs-nummer | Wachstums-Faktor a | % BNC Ausbeute | Selektions-faktor p | Taxonomie |
|---|---|---|---|---|
| DSM 1418 | 2.375 | 13 | 1,3 | Corynebacterium |
| DSM 1419 | 1.060 | 28 | 2,8 | " |
| DSM 1423 | 2.940 | 29 | 2,9 | " |
| DSM 1424 | 1.220 | 21 | 2,1 | " |
| DSM 1425 | 2.600 | 34 | 3,4 | " |
| DSM 1426 | 1.660 | 34 | 3,4 | " |
| DSM 1427 | 1.800 | 35 | 3,5 | " |
| DSM 1428 | 1.360 | 38 | 3,8 | " |
| DSM 1429 | 1.220 | 29 | 2,9 | " |
| DSM 1430 | 1.040 | 32 | 3,2 | " |
| DSM 1431 | 1.560 | 27 | 2,7 | " |
| DSM 1432 | 1.280 | 25 | 2,5 | " |

**Beispiel 2**

Der Wildstamm CBS 660.77 wird gemäß den Angaben des EP 004 913 zur Defektmutante CBS 437.77 umgewandelt. Dieser Mutantenstamm wird gemäß den nachfolgenden Angaben einer erneuten Mutation und Selektion unterworfen.

A Mutation

Der Stamm CBS 437.77 wird zunächst in folgender Nährlösung bei 30°C vermehrt (Nährlösung A): 0,8 % Pepton, 0,9 % Hefeextrakt, 0,3 % Glukose, 0,06 % Tween 80 (Polyoxyäthylensorbitanmonooleat), 0,06 % Cholesterin, $p_H$ 7,2. Nach Erreichen der logarithmischen Wachstumsphase werden die Zellen des Stammes unter sterilen Bedingungen abzentrifugiert, 2 mal mit sterilem 0,1 m Phosphatpuffer ($p_H$ 6,5) gewaschen, in dem gleichen Puffer resuspendiert und unter dem Mikroskop die Zelldichte auf $10^8$ Zellen/ml eingestellt. 8 ml dieser Zellsuspension werden in eine Petischale überführt und 90 Sekunden unter der UV-Lampe bestrahlt (Abstand 30 cm, UV-Bestrahlungslampe der Firma E. Schütt Jun., Göttingen). Die solchermaßen behandelte Zellsuspension wird anschließend in frischer Nährlösung A resuspendiert und bebrütet.

B Selektion und Isolierung der gewünschten Defektmutanten Penicilline- und "replica-Stempel"-Methode

Die wie unter A behandelte Kultur wird ca. 72 Stunden bei 30°C auf der Schüttelmaschine inkubiert bis zu einem Zelltiter von $> 10^9$ Zellen pro ml Kulturlösung.

0,1 ml dieser Zellsuspension in 10 ml folgender Nährlösung übertragen (Nährlösung B): 0,05 % $NaH_2PO_4$, 0,20 % $K_2HPO_4$, 0,05 % $MgSO_4 \cdot 7H_2O$, 0,02 % $CaCl_2 \cdot 2H_2O$, 0,005 % $MnSO_4 \cdot 4H_2O$, 0,005 % $(Fe)_2SO_4 \cdot 7H_2O$, 0,10 % $(NH_4)SO_4$, 0,0001 % Biotin, 0,10 % Tween 80 und 0,10 % BNC. Nach 18-stündigem Schütteln bei 30°C wird mit frischer, vorgewärmter Nährlösung derselben Zusammensetzung auf ca. $10^6$ Zellen pro ml verdünnt, 1000 IU Penicillin G zugesetzt und weitere 7 Stunden bei 30°C inkubiert. Dann wird das Antibiotikum durch Abzentrifugieren der Zellen mit Waschen mit steriler, physiologischer Kochsalzlösung entfernt und die Zellen in obengenanntem Medium mit 0,2 % Cholesterin anstelle von BNC für weitere 3 Tage inkubiert. Die Penicillinbehandlung wird ein weiteres Mal wiederholt und die Zellsuspension anschließend auf Nährmedium A plus 1,6 % Agar ausplattiert. Nach dem Vermehren der Zellen zu sichtbaren Kolonien wird mit Hilfe der "replica-Stempel-Technik" auf Nährmedium B plus 1,6 % Agar abgestempelt und diejenigen Kolonien selektiert und als Stammkulturen angelegt, die auf diesem Medium nicht oder nur kaum noch zu wachsen vermögen.

C Auswahl der aktivsten Defektmutanten

Nach dem unter B angegebenen Trenn- und Selektionsverfahren wurden ca. 90 Stämme isoliert.

Zur Auswahl der aktivsten BNC-anhäufenden Mutanten wurden sämtliche Stämme nochmals in Submerskultur getestet. Die Anzucht erfolgte in Reagenzgläsern mit je 2 ml Nährlösung A. Nach 24 Stunden bei 30°C auf dem "Roler Tube" wurden 0,1 % Tween 80 und 0,1 % Cholesterin zugegeben und nach weiteren 96 Stunden die Transformationsprodukte durch Extraktion und Dünnschichtchromatographie analysiert.

**Beispiel 3**

A BNC-Ausbeuten der aktivsten Mutantenstämme

1. BNC-Ausbeuten des Stammes DSM 1444

Der Stamm DSM 1444 wurde im 500 ml Erlenmeyerkolben mit 100 ml Nährlösung folgender Zusammensetzung gezüchtet: 0,8 % Pepton, 0,9 % Hefeextrakt, 0,3 % Glukose, 0,06 % Tween 80 (Polyoxyäthylensorbitanmonooleat), 0,06 % β-Sitosterin bzw. Cholesterin, $p_H$ 7,2. Die Kultur wurde auf der Schüttelmaschine (Schüttelfrequenz 150 UpM) bei 30°C für 62 Stunden vorgezüchtet, anschließend 0,1 % BRIJ 35 (Polyoxyäthylenmonolauryläther) und 0,1 % Cholesterin zugegeben und für weitere 120 Stunden bebrütet. Nach Abbruch der Kulturen werden Proben genommen und diese extrahiert und dünnschichtchromatographisch analysiert.

Bezogen auf die eingesetzte Substratmenge wurden 35 % 3-Oxo-pregna-1,4-dien-20-carbonsäure und 4 % 3-Oxo-pregna-4-en-20-carbonsäure und geringe Mengen von Androst-1,4-dien-3,17-dion gebildet.

2. BNC-Ausbeuten des Stammes DSM 1445

Unter den gleichen Bedingungen wie oben angegeben bildete der Stamm DSM 1445 folgende Produkte: 42 % 3-Oxo-pregna-1,4-dien-20-carbonsäure, 9 % 3-Oxo-pregna-4-en-20-carbonsäure und Spuren von AD und ADD.

Im Rahmen der Erfindung besonders geeignete Mikroorganismen-Wildstämme und Mutantenstämme, ihre Hinterlegungsstelle und ihr Hinterlegungsdatum sind nachfolgend genannt:

CBS = Centraalbureau voor Schimmelcultures, Baarn, Niederlande

DSM = Deutsche Sammlung von Mikroorganismen, Grisebachstraße 8, D-3400 Göttingen

**Wildstämme**

CBS 660.77 27.12.1977
DSM 1418 8. 1.1979
DSM 1419 8. 1.1979
DSM 1421 8. 1.1979
DSM 1423 8. 1.1979
DSM 1424 8. 1.1979
DSM 1425 8. 1.1979
DSM 1426 8. 1.1979
DSM 1427 8. 1.1979
DSM 1428 8. 1.1979
DSM 1429 8. 1.1979
DSM 1430 8. 1.1979
DSM 1431 8. 1.1979
DSM 1432 8. 1.1979

**Mutantenstämme**

CBS - 437.77 31. 8.1977
DSM 1435 8. 1.1979
DSM 1437 8. 1.1979
DSM 1439 8. 1.1979
DSM 1442 8. 1.1979
DSM 1443 8. 1.1979
DSM 1444 8. 1.1979
DSM 1445 8. 1.1979

**Patentansprüche**

1. Verfahren zur Herstellung von Mikroorganismen-Defektmutanten, die zur technischen Gewinnung von 17-C-Steroidα-propionsäureverbindungen, insbesondere zur Herstellung von 3-Oxo-pregn-4-en-20-carbonsäure (Δ-4 BNC) und/oder 3-Oxo-pregna-1,4-dien-20-carbonsäure (Δ-1,4 BNC), aus 17-C-Seitenketten-Steroidsubstraten, z.B. aus Sterinverbindungen tierischen oder pflanzlichen Ursprungs, durch wenigstens weitgehend selektiven Seitenkettenabbau unter aeroben Bedingungen auch in Abwesenheit von den Steroidringabbau und/oder das Wachstum hemmenden Inhibitoren befähigt sind, dadurch gekennzeichnet, daß man

1. einen Mikroorganismen-Wildstamm isoliert und züchtet, der zum Wachstum auf Sterinverbindungen als alleiniger Kohlenstoffquelle befähigt ist,

2. den Wildstamm einer an sich bekannten Mutationsbehandlung unterwirft,

3. die Mutantenpopulation auf einem Trennmedium züchtet, auf dem die 17-C-Steroid-α-propionsäureverbindungen liefernden Mutanten praktisch nicht wachsen, während die unerwünschten begleitenden Mutantenstämme wachsen und hierdurch bzw. während ihres Wachstums abgetötet werden und

4. den verbliebenen Rest der Mutantenstämme auf 17-C-Seitenkettensterinen züchtet und dabei die Defekt-Mutantenstämme mit optimaler Produktion an 17-C-Steroid-α-propionsäureverbindungen isoliert,

wobei man in der Stufe zu 1. einen Wildstamm isoliert und züchtet, der beim aeroben Wachstum auf Sterinverbindungen in Gegenwart von Inhibitoren für den enzymatischen Ringabbau der Sterinverbindungen wenigstens anteilsweise 17-C-Steroid-α-propionsäureverbindungen liefert.

2. Verfahren nach Anspruch 1, dadurch gekennzeichnet, daß man in der Stufe zu 1. solche Wildstämme isoliert und züchtet, die beim Wachstum auf Sterinverbindungen mit gesättigten oder ungesättigten Alkylresten in 17-C- mit 8 bis 10 C-Atomen unter Standardbedingungen eine Ausbeute von 17-C-Steroid-α-propionsäureverbindungen von wenigstens 5 Gew.-%, vorzugsweise von wenigtens 10 Gew.-% - bezogen auf die im Standardversuch eingesetzte(n) Sterinverbindung(en) - liefern.

3. Verfahren nach Ansprüchen 1 und 2, dadurch gekennzeichnet, daß man in der Stufe zu 1. Mikroorganismenwildstämme isoliert und züchtet, die beim Wachstum auf Sterinverbindungen mit gesättigten oder ungesättigten Alkylresten in 17-C- mit 8 bis 10 C-Atomen unter Standardbedingungen eine selektive Abbauleistung gemäß der allgemeinen Formel

$$I = a \cdot 10^{p}$$

ergeben, worin a der Wachstumsfaktor und p der Selektivitätsfaktor des Wildstammwachstums sind und der Selektivindex I den Zahlenwert von wenigstens 1, vorzugsweise von wenigstens 2 und insbesondere den Wert von wenigstens 20 beträgt.

4. Verfahren nach Ansprüchen 1 bis 3, dadurch gekennzeichnet, daß Mikroorganismenwildstämme isoliert und gezüchtet werden, deren Wachstumsfaktor a unter Standardbedingungen wenigstens 0,2, vorzugsweise wenigstens 1, und deren Selektivitätsfaktor p unter Standardbedingungen wenigstens 0,5, vorzugsweise wenigstens 1, betragen.

5. Verfahren nach Ansprüchen 1 bis 4, dadurch gekennzeichnet, daß man in der Stufe zu 1. Wildstämme isoliert und züchtet, die den 17-C-Seitenkettenabbau einem Ringabbau gegenüber bevorzugen.

6. Verfahren nach Ansprüchen 1 bis 5, dadurch gekennzeichnet, daß die Mutationsbehandlung der Wildstämme gemäß der Stufe zu 2. unter solchen Bedingungen von Konzentration und Einwirkzeit des mutagenen Agens durchgeführt wird, daß die Ausgangspopulation der Mikroorganismen durch die mutagene Behandlung zu 10 bis 99,999 % inaktiviert wird, wobei vorzugsweise mit einer Abtötungsrate von 90 bis 99,99% gearbeitet wird.

7. Verfahren nach Ansprüchen 1 bis 6, dadurch gekennzeichnet, daß man als Wildstämme einen der folgenden Mikroorganismen einsetzt: DSM 1418, DSM 1419, DSM 1423, DSM 1424, DSM 1425, DSM 1426, DSM 1427, DSM 1428, DSM 1429, DSM 1430, DSM 1431, DSM 1432.

8. Verfahren zur Herstellung von 17-C-Steroid-α-propionsäureverbindungen durch mikrobiellen Seitenkettenabbau an 17-C-Seitenketten-Steroidsubstraten, insbesondere solchen tierischen und/oder pflanzlichen Ursprungs, bei dem man

a) Mikroorganismen-Defektmutanten, die auch in Abwesenheit von den Steroidringabbau und/oder das Wachstum hemmenden Inhibitoren Steroidverbindungen mit dem 17-C-α-propionsäurerest liefern, in einem wäßrigen Nährmedium unter aeroben Bedingungen in Gegenwart des Steroidsubstrats unter Anreicherung der 17-C-Steroid-α-propionsäureverbindungen in der Fermentationsbrühe züchtet und

b) die gebildeten 17-C-Steroid-α-propionsäureverbindungen isoliert,

dadurch gekennzeichnet, daß man mit Defektmutanten als Mikroorganismen arbeitet, die nach dem Verfahren gemäß einem oder mehreren der Ansprüche 1 bis 7 hergestellt worden sind.

9. Verfahren nach Anspruch 8, dadurch gekennzeichnet, daß man mit Defektmutanten arbeitet, die bei Züchtung auf Cholesterin oder Sitosterin unter Standardbedingungen eine Mindestausbeute an Δ-4- und/oder Δ-1,4 BNC von 15 %, vorzugsweise von wenigstens 30 % und bei der Züchtung auf Cholesterin insbesondere eine Ausbeute von wenigstens 50 % - jeweils bezogen auf das Gewicht der eingesetzten Sterinverbindung - liefern.

10. Verfahren nach Ansprüchen 8 und 9, dadurch gekennzeichnet, daß man mit Defektmutanten von Achromobacter, Arthrobacter, Bacillus, Brevibacterium, Corynebacterium, Flavobacterium, Microbacterium, Mycobacterium, Nocardia, Protaminobacterium, Serratia oder Streptomyces arbeitet.

11. Verfahren nach Ansprüchen 8 bis 10, dadurch gekennzeichnet, daß man das zu transformierende Steroidsubstrat der Reaktionszone wenigstens weitgehend kontinuierlich zusetzt.

12. Verfahren nach Ansprüchen 8 bis 11, dadurch gekennzeichnet, daß man mit einem der folgenden Defektmutanten arbeitet: DSM 1435, DSM 1437, DSM 1439, DSM 1442, DSM 1443, DSM 1444 und DSM 1445.

## Claims

1) A process for preparing defective microorganism mutants capable of commercially recovering 17-C steroid α-propionic acid compounds, more specifically of producing 3-oxo-pregn-4-ene-20-carboxylic acid (Δ-4 BNC) and/or 3-oxo-pregna-1,4-diene-20-carboxylic acid (Δ-1,4 BNC) from 17-C side chain steroid substrate, e.g. from sterol compounds of animal or plant origin, by an at least to a far extent selective degradation of side chains under aerobic conditions even in the absence of inhibitors inhibiting the degradation of the steroid ring and/or the growth,

characterized by

1. isolating and cultivating a microorganism wild strain capable of growing on sterol compounds as the sole carbon source;

2. subjecting the wild strain to a per se known mutation treatment;

3. cultivating the mutant population on a separating medium on which the mutants providing 17-C steroid α-propionic acid compounds do virtually not grow, while the undesired accompanying mutant strains do grow and are killed thereby or during the growth thereof, respectively; and

4. cultivating the remaining mutant strains on 17-C side chain sterols and isolating the defective mutants yielding optimum production of 17-C steroid α-propionic acid compounds,

wherein the wild strain being isolated and cultivated in the stage 1. is a wild strain which upon aerobic growth on sterol compounds in the presence of inhibitors for the enzymatic ring degradation of the sterol compounds does at least portion-wise yield 17-C steroid α-propionic acid compounds.

2.) The process according to claim 1, characterized in that in the stage 1. such wild strains are isolated and cultivated which upon growth on sterol compounds having saturated or unsaturated alkyl moieties comprising 8 to 10 carbon atoms in 17-C under standard conditions provide a yield of 17-C steroid α-propionic acid compounds of at least 5% by weight, and preferably at least 10% by weight, based on the sterol compound(s) employed in the standard experiment.

3.) The process according to claims 1 and 2, characterized in that in the stage 1. microorganism wild strains

are isolated and cultivated which upon growth on sterol compounds having saturated or unsaturated alkyl moieties comprising 8 to 10 carbon atoms in 17-C under standard conditions provide a selective degradation output according to the general formula

$I = a \cdot 10^p$

wherein a is the growth factor and p is the selectivity factor of the wild strain growth and the selectivity index I has a numerical value of at least 1, and preferably of at least 2, and more particularly the value of at least 20.

4.) The process according to claims 1 to 3, characterized in that microorganism wild strains are isolated and cultivated the growth factor a of which under standard conditions is at least 0.2, and preferably at least 1, and the selectivity factor p of which under standard conditions is at least 0.5, and preferably at least 1.

5.) The process according to claims 1 to 4, characterized in that in the stage 1. microorganism wild strains are isolated and cultivated which prefer the 17-C side chain degradation over a ring degradation.

6.) The process according to claims 1 to 5, characterized in that the mutation treatment according to the stage 2. is carried under such conditions of concentration and time of action of the mutagenic agent that the initial population of the microorganisms is inactivated by the mutagenic treatment to an extent of from 10 to 99.999%, a killing rate of from 90 to 99.99% being preferred.

7.) The process according to claims 1 to 6, characterized in that one of the following microorganisms is employed as the wild strains: DSM 1418, DSM 1419, DSM 1423, DSM 1424, DSM 1425, DSM 1426, DSM 1427, DSM 1428, DSM 1429, DSM 1430, DSM 1431, DSM 1432.

8.) A process for preparing 17-C steroid α-propionic acid compounds by microbial degradation of the side chains of 17-C side chain steroid substrates, more specifically those of animal and/or plant origin, wherein

a) microorganism defective mutants which provide steroid compounds having the 17-C steroid α-propionic acid moiety even in the absence of inhibitors inhibiting the degradation of the steroid ring and/or the growth are cultivated in an aqueous medium under aerobic conditions in the presence of the steroid substrate to accumulate the 17-C steroid α-propionic acid compounds in the fermentation broth and

b) the 17-C steroid α-propionic acid compounds are isolated,

characterized in that defective mutants prepared according to one or more of the claims 1 to 7 are employed as the microorganisms.

9.) The process according to claim 8, characterized in that defective mutants are employed which when cultivated on cholesterol or sitosterol under standard conditions provide a minimum yield of Δ-4 BNC and/or Δ-1,4 BNC of 15%, and preferably of at least 30%, and, more specifically, when cultivated on cholesterol provide a yield of at least 50% - each based on the weight of the sterol compound employed.

10) The process according to claims 8 and 9, characterized in that defective mutants of Achromobacter, Arthrobacter, Bacillus, Brevibacterium, Corynebacterium, Flavobacterium, Microbacterium, Mycobacterium, Nocardia, Protaminobacterium, Serratia or Steptomyces are employed.

11.) The process according to claims 8 to 10, characterized in that the steroid substrate to be transformed is at least mostly continuously added to the reaction zone.

12.) The process according to claims 8 to 11, characterized in that one of the following defective mutants is employed: DSM 1435, DSM 1437, DSM 1439, DSM 1442, DSM 1443, DSM 1444 and DSM 1445.

**Revendications**

1°) Procédé pour l'obtention de mutants défectueux (déficients) de micro-organismes, qui sont aptes à la production industrielle de composés stéroïdes a reste α-propionyle en $C_{17}$, en particulier à la production d'acide 3-oxo-prégn-4-ène-20-carboxylique (Δ-4 BNC) et/ou d'acide 3-oxo-prégna-1,4-diène-20-carbo-xylique (Δ-1,4 BNC) à partir de substrats stéroïdes à chaîne latérale en $C_{17}$, par exemple à partir de composés stéroliques d'origine animale ou végétale, par dégradation, au moins dans une large mesure sélective, de la chaîne latérale, dans des conditions aérobies, même en absence d'inhibiteurs inhibant la dégradation du noyau stéroïde et/ou la croissance, caractérisé en ce que:

1. on isole et on cultive une souche sauvage de microorganismes qui est capable de croissance sur des composés stéroliques en tant que source unique de carbone,

2. on soumet la souche sauvage à un traitement mutagène connu en soi,

3. on cultive la population de mutants sur un milieu sélectif, sur lequel les mutants produisant des composés à reste α-propionyle en $C_{17}$ ne se développent pratiquement pas, tandis que les souches mutantes indésirables qui les accompagnent se développent et sont de ce fait tuées ou le sont pendant leur croissance, et

4. on cultive le reste des souches mutantes sur des stérols à chaîne latérale en $C_{17}$, et on isole par ce moyen les souches de mutants défectueux ayant une production optimale des composés stéroïdes à reste α-propionyle en $C_{17}$,

ce par quoi, dans l'étape I, on isole et on cultive une souche sauvage qui produit, lors de la croissance aérobie sur des composés stéroliques, en présence d'inhibiteurs de la dégradation enzymatique du noyau des composés stéroliques, au moins des proportions de composés stéroïdes à reste α-propionyle en $C_{17}$.

2°) Procédé selon la revendication 1, caractérisé en ce que, dans l'étape I, on isole et on cultive des souches sauvages qui donnent, dans des conditions standard, lors de la croissance sur des composés stéroliques à

reste alkyle en $C_{17}$, saturés ou non saturés contenant de 8 à 10 atomes de carbone, un rendement en composés stéroïdes à reste α-propionyle en $C_{17}$ d'au moins 5 % en poids, de préférence d'au moins 10 % en poids, par rapport au(x) composé(s) stérolique(s) utilisé(s) dans l'essai normalisé.

3°) Procédé selon les revendications 1 et 2, caractérisé en ce que, dans l'étape I, on isole et on cultive des souches sauvages de micro-organismes qui manifestent, dans des conditions standard, lors de la croissance sur des composés stéroliques à restes alkyle en $C_{17}$, saturés ou non saturés, contenant de 8 à 10 atomes de carbone, une capacité de dégradation sélective selon la formule générale:

$$I = a \cdot 10^{p}$$

dans laquelle a est le facteur de croissance et p est le facteur de sélectivité de la croissance de la souche sauvage, et l'indice de sélectivité I s'élève à la valeur numérique d'au moins 1, de préférence d'au moins 2, et en particulier à la valeur d'au moins 20.

4°) Procédé selon l'une quelconque des revendications 1 à 3, caractérisé en ce que l'on isole et cultive des souches sauvages de micro-organismes dont le facteur de croissance a s'élève dans des conditions standard, à au moins 0,2, de préférence à au moins 1, et dont le facteur de sélectivité p s'élève, dans des conditions standard, à au moins 0,5, de préférence à au moins 1.

5°) Procédé selon l'une quelconque des revendications 1 à 4, caractérisé en ce que, dans l'étape I, on isole et on cultive des souches sauvages qui préfèrent la dégradation de la chaîne latérale en $C_{17}$ à une dégradation du noyau.

6°) Procédé selon l'une quelconque des revendications 1 à 5, caractérisé en ce que l'on effectue le traitement mutagène des souches sauvages, selon l'étape II dans des conditions de concentration et de durée d'action de l'agent mutagène telles que la population initiale des micro-organismes est inactivée à 10-99,999 % par le traitement mutagène, en opérant de préférence avec un taux de mortalité de 90 à 99,999 %.

7°) Procédé selon l'une quelconque des revendications 1 à 6, caractérisé en ce que l'on utilise, en tant que souches sauvages, l'un des microorganismes suivants: DSM 1418, DSM 1419, DSM 1423, DSM 1424, DSM 1425, DSM 1426, DSM 1427, DSM 1428, DSM 1429, DSM 1430, DSM 1431, DSM 1432.

8°) Procédé pour la production de composés stéroïdes à reste α-propionyle en $C_{17}$, par dégradation microbienne de la chaîne latérale de substrats stéroïdes à chaîne latérale en $C_{17}$, en particulier ceux d'origine animale et/ou végétale, dans lequel

a) on cultive dans un milieu nitritif aqueux, dans des conditions aérobies en présence du substrat stéroïde, des mutants défectueux de micro-organismes qui produisent des composés stéroïdes comportant le reste -propionyle en $C_{17}$, même en absence d'inhibiteurs inhibant la dégradation du noyau stéroïde et/ou la croissance, avec accumulation dans le bouillon de fermentation de composés stéroïdes à reste α-propionyle en $C_{17}$, et

b) on isole les composés stéroïdes à reste α-propionyle en $C_{17}$ formés,

caractérisé en ce que l'on opère en utilisant, en tant que micro-organismes, des mutants déficients qui ont été obtenus par le procédé selon une ou plusieurs des revendications 1 à 7.

9°) Procédé selon la revendication 8, caractérisé en ce que l'on opère en utilisant des mutants déficients qui donnent, lors de culture sur cholestérol ou sitostérol, dans des conditions standard, un rendement minimal en Δ-4 BNC et/ou Δ-1,4 BNC de 15 %, de préférence d'au moins 30 %, et dans la culture sur cholestérol en particulier, un rendement d'au moins 50 %, chaque fois par rapport au poids du composé stérolique utilisé.

10°) Procédé selon les revendications 8 et 9, caractérisé en ce que l'on opère avec des mutants déficients d'Achromobacter, Arthrobacter, Bacillus, Brevibactérium, Corynebactérium, Flabobactérium, Microbacterium, Mycobactérium, Nocardia, Protaminobactérium, Serratia ou Streptomyces.

11°) Procédé selon l'une quelconque des revendications 8 à 10, caractérisé en ce que l'on introduit dans la zone de réaction le substrat stéroïde à transformer, au moins dans une large mesure en continu.

12°) Procédé selon l'une quelconque des revendications 8 à 11, caractérisé en ce que l'on opère avec un des mutants déficients suivants: DSM 1435, DSM 1437, DSM 1439, DSM 1442, DSM 1443, DSM 1444 et DSM 1445.